(19) Europäisches Patentamt
European Patent Office
Office européen des brevets

(11) EP 4 598 059 A1

(12) EUROPEAN PATENT APPLICATION

(43) Date of publication:
06.08.2025 Bulletin 2025/32

(21) Application number: 25154843.4

(22) Date of filing: 30.01.2025

(51) International Patent Classification (IPC):
H04R 25/00 (2006.01)

(52) Cooperative Patent Classification (CPC):
H04R 25/70; H04R 25/505

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 05.02.2024 EP 24155836

(71) Applicant: Interacoustics A/S
5500 Middelfart (DK)

(72) Inventors:
• LAUGESEN, Søren
5500 Middelfart (DK)

• HOCKLEY, Neil Spencer
3018 Berne (CH)
• SANTURETTE, Sébastien
2765 Smørum (DK)
• ZAAR, Johannes
2765 Smørum (DK)
• JONES, Gary
2765 Smørum (DK)

(74) Representative: Demant
Demant A/S
Kongebakken 9
2765 Smørum (DK)

(54) PRESCRIBING HEARING AID FEATURES FROM DIAGNOSTIC MEASURES

(57) A method of setting signal processing parameters of a hearing aid is presented. The method comprises determining Audible Contrast Threshold (ACT) data of at least one ear of a hearing aid user, determining an audiogram of the hearing aid user, determining an age parameter of the hearing aid user, determining signal processing parameter settings for a hearing aid for use by the hearing aid user according to a prescription protocol in dependence of said ACT data, audiogram, and age parameter, setting signal processing parameters of the hearing aid.

FIG. 1

EP 4 598 059 A1

**Description**

TECHNICAL FIELD

The present application relates to the field of hearing aids.

SUMMARY

The present application relates to a method of setting signal processing parameters of a hearing aid.
The present application further relates to a hearing aid fitting system.
The present application further relates to a data processing system comprising a processor and program code means.
The present application further relates to a computer program comprising instructions.
Hearing loss manifests itself in several ways, the most obvious of which are loss of audibility and hearing-in-noise difficulties. Today, when fitting hearing aids to alleviate hearing loss, evidence-based fitting rules only exist for amplification, or gain, addressing only the first problem. Thus, for more than 100 years, hearing aids have been fitted from only one diagnostic measure: the pure-tone audiogram, which describes the softest sounds a given client can hear at various frequencies. This has been successful in terms of prescribing amplification to solve the client's audibility issues. However, as already mentioned, a hearing loss is much more than lost audibility; typically, the ability to hear in noise is also negatively affected, but to various degrees that are not necessarily related to the audiometric hearing loss.
To improve on the hearing aid user's hearing-in-noise problems, modern hearing aids include various help-in-noise features, such as directional microphones (beamformers) and spectralsubtraction-type noise reduction or more generally post-filtering. In recent years, these help-in-noise features have become much more powerful, which means that there is a muchimproved potential to fit the settings of the help-in-noise features to the individual based on their specific needs. This potential is so far untapped because of a lack of clinically viable diagnostic measures to predict a given client's aided hearing-in-noise performance, which could form the basis of a prescription of help-in-noise features. The term "clinically viable" is many-faceted, where some of the key factors are time-efficiency and simplicity of the physical and technical setup. Instead, the usual practice is to, as a starting point, prescribe middle-of-the-road default settings of these features to all hearing aid users, which will be optimal for the middle-of-the-road clients, but less so for those with hearing-in-noise performance deviating from the average. This is a lost opportunity, particularly for those hearing aid users who struggle disproportionately in noise because these users would benefit substantially from the strongest possible settings of the help-in-noise features, and in particular from a setting that engages the help-in-noise features in the - relatively simple - sound environments in which these people already have problems.
Today, hearing-care professionals are encouraged (or in some countries even required) to do speech-in-noise testing, and while potentially useful for prescribing help-in-noise features, there is no clear guidance as to how to use the results of the speech-in-noise tests within the hearing aid fitting process. Poor initial fitting would potentially also result in some users giving up on hearing aids and returning their hearing aids within the trial period.
Other hearing aid processing features are also lacking an evidence-based fitting approach, e.g., Brightness and Soft-gain.
Accordingly, there is a need for an improved fitting approach taking into account speech-in-noise testing.

A method:

In an aspect of the present application, a method of setting signal processing parameters of a hearing aid is provided.
Setting may refer to fitting/adjusting the signal processing parameters of the hearing aid to the needs/requirements of the hearing aid user.
The method may comprise determining Audible Contrast Threshold (ACT) data of at least one ear of a hearing aid user.
For example, the ACT data may be determined by providing a stimulus into one or both ears of the hearing aid user and receiving a response from the user. For example, the user may provide a response via a physical response button, a user interface, electrophysiologically (with use of one or more electrodes attached to the head), etc.
For example, a spectro-temporal modulation STM detection threshold (and eventually an ACT) is experimentally found by adaptively varying the degree of modulation in a stimulus, which is delivered through headphones or insert earphones to the ears of the user. The user is asked to respond to "target" stimuli with modulations while comparing these to unmodulated "reference" stimuli. The threshold is then the smallest degree of modulation that the user can detect. The general thinking is that if a person is good at the ACT test (or equivalently STM detection), then they will also be good at picking out speech from background noise even when there is very little contrast between speech and background noise.

Vice versa, a person with poor ACT/STM will need a larger contrast between speech and background noise to understand the speech. Using ACT/STM to estimate speech-in-noise ability has the further advantage that the testing is not using language-specific speech material but relies on artificial stimuli. In this way, ACT/STM can be used with anyone in any country irrespective of language background.

Several studies have shown that the ACT test has considerable predictive power to account for aided speech-in-noise performance in realistic listening conditions. The ACT test is a clinically valid implementation of the STM test, and there is additional research evidence to back up the relationship between STM (and therefore ACT) and speech-in-noise performance [1]. Thus, ACT may be used in the hearing aid fitting process to estimate a user's aided speech-in-noise performance, which in turn allows one to prescribe the hearing aid's help-in-noise features to the user. Also, binaural measurements of ACT may be used to prescribe the use of bilateral beamformer technologies.

Accordingly, ACT can be conveniently measured right after measuring an audiogram, i.e., when the hearing aid user is already wearing headphones or insert earphones and has a response button in hand. Thus, the ACT data may be available very early in the fitting process.

The method may comprise determining an audiogram of the hearing aid user.

The method may comprise determining an age parameter of the hearing aid user.

Age parameter may refer to the age of the hearing aid user.

The method may comprise determining signal processing parameter settings for a hearing aid for use by the hearing aid user.

The signal processing parameter settings may be determined according to a prescription protocol in dependence of at least said ACT data.

The signal processing parameter settings may be determined according to a prescription protocol in dependence of at least said audiogram.

The signal processing parameter settings may be determined according to a prescription protocol in dependence of at least said age parameter.

The signal processing parameter settings may be determined according to a prescription protocol in dependence of (taking into account) said ACT data, audiogram, and age parameter.

The method may comprise setting signal processing parameters of the hearing aid.

Thereby, an improved fitting approach is provided which takes into account speech-in-noise testing.

The ACT data may comprise an ACT value.

The ACT value may be a normalized contrast level (nCL) value.

0 dB nCL may correspond to a median performance of a test group of normal hearing test subjects.

16 dB nCL may correspond to the physically maximal degree of spectro-temporal modulation, that is, 0 dB Full Scale (FS).

In this way, 0 dB nCL corresponds to normal performance, while positive dB nCL values indicate some degree of contrast loss and negative dB nCL values indicate better-than-normal performance. Also, in alignment with the audiogram procedure, where testing may be capped at -10 dB HL, the normalized Contrast Level is not adapted beyond -4 dB nCL, two steps below 0 dB nCL. In this way, the Contrast Level (dB nCL) scale used for ACT quantifies contrast loss in the same way as the Hearing Level (dB HL) scale quantifies audibility loss.

The ACT value may be one (combined) value for both of the ears.

The ACT value may be one value for each of the ears of the hearing aid user.

The step of determining ACT data may comprise estimating ACT data of a least one of the ears of the hearing aid user in an ACT test.

The step of determining ACT data may comprise receiving estimated ACT data of the hearing aid user from a database.

The step of setting signal processing parameters of the hearing aid may comprise transmitting said determined signal processing parameter settings to the hearing aid.

The step of determining an audiogram of the hearing aid user may comprise estimating one or more Hearing Threshold Level (HTL) values from a measured audiogram of the hearing aid user.

The step of determining an audiogram of the hearing aid user may comprise receiving a measured audiogram or estimated HTL values from a database.

The step of determining an age parameter may comprise inputting an age parameter of the hearing aid user.

The step of determining an age parameter may comprise receiving an age parameter of the hearing aid user from a database.

The step of determining ACT data of the hearing aid user may comprise pre-processing said ACT data to reduce dimensionality.

The step of determining an audiogram of the hearing aid user may comprise pre-processing said audiogram to reduce dimensionality.

The step of pre-processing said audiogram may comprise estimating HTL values at a range of audiometric frequencies.

The step of pre-processing said audiogram may comprise estimating a four-frequency average value, e.g. a Pure-Tone Average (PTA) value.

For example, the HTL values at a range of audiometric frequencies and specific to left and right ears of a hearing aid user can be used to compute a four-frequency average across both ears, typically denoted the PTA or PTA4.

The step of setting signal processing parameters of the hearing aid according to a prescription protocol may comprise defining a multitude of prescription levels.

Each of the multitude of prescription level may comprise pre-set signal processing parameters. For example, the prescription levels may define help-in-noise settings to aid a hearing aid user's hearing-in-noise problems going from 'very high' (much aid) to 'very low' (low aid), such as from 'very high' to 'high' to 'medium' to 'low' to 'very low', or such as from 1 to 5. Defining a multitude of prescription levels may comprise determining a linear regression model as prescription protocol for defining said multitude of prescription levels in dependence of said ACT value, audiogram, and age parameter.

For example, the linear regression model may be estimated based on pre-determined ACT data, audiograms, and/or age parameters from a multitude of users. For example, the pre-determined ACT data, audiograms, and/or age parameters from a multitude of users may be determined/measured at a time prior to determining the linear regression model.

For example, the audiograms may be PTA such as a four-frequency average, PTA4, across two ears of each of the users.

In other words, coefficients of the linear regression model may be estimated/determined based on said pre-determined ACT data, audiograms, and/or age parameters from said multitude of users (e.g., a test population). For example, the coefficients may comprise an ACT value specific coefficient ($k_{ACT}$), a PTA value specific coefficient ($k_{PTA}$), and/or an age parameter specific coefficient ($k_{AGE}$).

For example, said coefficients may comprise means of the regression coefficients, and/or standard errors of the regression coefficients.

For example, the values of the coefficients may be selected from the output of the regression model in various ways:

- the mean for each coefficient can be selected,
- values greater or less than the mean can be selected,
- values greater or less than the mean can be selected. For example, the selection may be finetuned by user input (e.g., user satisfaction), device return rates, refitting rates, etc. When selecting values of coefficients greater or less than the means, the selection may be counterbalanced so as to keep the mean and variance of the model's predictions for the test population unchanged relative to a baseline model using the means of the regression coefficients.

The linear regression model may take the form:

$$SRT = k_{ACT} \times ACT + k_{PTA} \times PTA + k_{AGE} \times Age + C$$

Where SRT is the estimated speech reception threshold (SRT) value, $k_{ACT}$ is the ACT value specific coefficient, $k_{PTA}$ is the PTA value specific coefficient, $k_{AGE}$ is the age parameter specific coefficient, ACT is the determined ACT value of at least one ear of the hearing aid user, PTA is the determined PTA value of the hearing aid user, Age is the determined age parameter of the hearing aid user, and C is a constant.

The multitude of prescription levels may be separated by thresholds.

The thresholds may be SRT thresholds.

A threshold may refer to SRT values defining a border between two prescription levels.

For example, a prescription level may refer to an interval/range of SRT values.

The linear regression model may estimate an SRT value of the hearing aid user.

The SRT value may be a combined value for both of the ears of the hearing aid user.

The SRT value may be estimated for each of the ears of the hearing aid user.

The linear regression model may be estimated based at least on pre-determined ACT data, audiograms, and age parameters.

The linear regression model may be estimated based at least on pre-determined signal processing parameters.

The one or more prescription levels may each comprise/define/determine a beamforming and/or noise reduction setting.

For example, the prescription levels may each determine the allowed angular range of nullsteering and null depth for an adaptive beamformer.

For example, the prescription levels may each determine the strength of the spectralsubtraction-type noise reduction.

For example, the prescription levels may each determine the strength of the postfiltering noise reduction.

For example, the prescription levels may each define the threshold values, in terms of, e.g., overall sound level and estimated signal-to-noise ratio, which determine when the help-in-noise features are activated.

The one or more prescription levels may each comprise/define/determine a high frequency gain (i.e., termed brightness) setting.

The one or more prescription levels may each comprise/define/determine a listening distance (i.e., termed soft gain) setting.

The one or more prescription levels may each comprise/define/determine a transient noise reduction setting.

The step of determining signal processing parameter settings for a hearing aid for use by the hearing aid user according to a prescription protocol in dependence of said ACT value, audiogram, and age parameter may comprise determining a value representative of one of said prescription levels.

The method may further comprise automatically setting SRT thresholds for said one or more prescription levels.

The method may comprise determining ACT data of both ears of the hearing aid user simultaneously.

For example, both ears of the hearing aid user may be provided with the same stimuli and ACT data indicating one combined ACT value may be determined. Alternatively, or additionally, the ears of the hearing aid user may be provided with different stimuli and ACT data indicating one combined ACT value may be determined.

The sound arriving at the eardrum is a mixture of (i) processed sound, picked up by the hearing-aid microphones, processed, and played through the hearing-aid receiver, and (ii) unprocessed sound, entering the ear canal from the outside. Hearing-aid processing can only manipulate the processed sound, whereas the unprocessed sound remains unchanged. Therefore, the physical effect of any hearing-aid processing feature on the sound representation at the eardrum, and thus its potential efficacy, depends largely on the relationship between these two components.

The relationship between processed and unprocessed sound is primarily driven by

1) The amount of amplification, determined by the audiogram via the gain prescription rule,
2) The input level, which affects the amount of amplification through dynamicrange compression, and
3) The amount of acoustic coupling, determined by how tightly the earpiece seals the ear canal and thus blocks unprocessed sound from entering.

The acoustic coupling therefore depends on the interaction between the size and type of earpiece selected for a given hearing aid user/patient (currently suggested by the fitting software based on the audiogram) and the patient's ear canals. The amount of acoustic coupling can be described by means of the real-ear occluded insertion gain (REOIG), which is defined as the difference between the real-ear occluded gain (REOG) and the real-ear unaided gain (REUG), thus describing the amount of attenuation (or negative gain) obtained through the earpiece (see [2]).

The acoustic coupling is intentionally limited by the inclusion of vents in the earpieces, which allow unprocessed sound to enter the ear canal, as very closed fittings lead to unpleasant problems with occlusion (e.g., in terms of own-voice perception, feelings of fullness, and discomfort) and are thus dispreferred by patients. The typical current approach is to use earpieces that are as open as possible whilst still allowing for appropriate control of the acoustical feedback path between the hearing-aid receiver and the hearing-aid microphones. However, it has been demonstrated that patients differ strongly regarding their tolerance for different levels of acoustic coupling (i.e., vent size and the resulting occlusion effects on own voice perception etc.) and that this tolerance can be predicted using a simple standard measure of tympanic membrane compliance (see [3]).

In the context of prescribing advanced help-in-noise features such as directionality (e.g., by directional microphone system) and noise reduction, the above-described aspects are relevant because the degree of ear openness stands in direct opposition to the amount of attainable improvements in signal-to-noise ratio (SNR), resulting in opposing interests (comfort vs. audiological benefit) that need to be balanced for an optimal fitting of hearing aids at an individual patient level. If a prediction of speech-in-noise performance is obtained (e.g., by the method of setting signal processing parameters of a hearing aid comprising determining ACT, an audiogram, and an age parameter, as described above) and converted into a specific need for SNR enhancement (e.g., by determining signal processing parameter settings for the hearing aid, as described above), it needs to be ensured that the desired SNR enhancement is indeed attainable with the selected hardware, ideally whilst balancing the need for SNR enhancement with the patients' tolerance for closed fittings.

The one or more prescription levels may each comprise/determine/define a degree of earmould venting.

For example, the degree of earmould venting may be determined and installed during fitting of the hearing aid, and in case of an active earmould vent, the degree may be set automatically. The one or more prescription levels may each comprise/determine/define a selection of instant-fit dome type.

For example, the instant-fit dome type may be selected during fitting of the hearing aid.

The method may further comprise, to estimate/determine an acoustic coupling.

The acoustic coupling may be estimated/determined in terms of a real-ear occluded insertion gain (REOIG) required to obtain the (needed) signal processing parameter settings of the hearing aid (i.e., the SNR enhancement).

For example, said signal processing parameter settings may be determined according to a prescription protocol in dependence of said ACT data, audiogram, and age parameter, as identified in above.

The method may further comprise, to identify a (correct) earpiece type and vent size that provides the required acoustic coupling.

For example, the required acoustic coupling may be the acoustic coupling estimated/determined in terms of the REOIG (as mentioned above).

For example, the earpiece and vent size may be based on average REOIG measures obtained for a plurality of earpieces with various ear canals.

It should be noted that the earpiece type and vent size alone are not perfect predictors of the attainable amount of acoustic coupling in terms of REOIG, as the earpiece interacts with the ear canal of the patient.

The method may further comprise, to counterbalance the earpiece and vent size identified (see above) with an individualized prediction of minimum acceptable equivalent vent size area based on a measured tympanic membrane compliance (see [3]), providing an upper limit for the acoustic coupling.

The method may further comprise, prescribing earpiece type and vent size based on the identified earpiece and vent size, and on the provided upper limit for the acoustic coupling.

In other words, the method may comprise one or more of the below steps:

1) Use the prediction of speech-in-noise performance based on ACT, PTA, and Age to estimate the patient's need for SNR enhancement

2) Estimate the acoustic coupling in terms of REOIG required to obtain the needed SNR enhancement identified in 1).

3) Identify the correct earpiece and vent size (e.g. based on average REOIG measures obtained for all earpieces with various ear canals\*) that provides the required acoustic coupling identified in 2).

4) Counterbalance the correct earpiece and vent size identified in 3) with an individualized prediction of minimum acceptable equivalent vent size area based on the measured tympanic membrane compliance, providing an upper limit for the acoustic coupling

5) Prescribe earpiece and vent size based on 3) and 4).

\* Relative to 3), it should be noted that the earpiece type and vent size alone are not perfect predictors of the attainable amount of acoustic coupling in terms of REOIG, as the earpiece interacts with the ear canal of the patient.

As mentioned, the earpiece type and vent size alone are not perfect predictors of the attainable amount of acoustic coupling in terms of REOIG, as the earpiece interacts with the ear canal of the patient. There are several ways to handle this problem.

Accordingly, the method may comprise one or more of the following:

- Use an average REOIG measure for each earpiece type, obtained with a large range of different ear canals
- Use a personalized REOIG measure for each earpiece type based on physiological aspects (e.g., head size, a measure of ear-canal diameter, etc.)
- Use a personalized REOIG measure for each earpiece type based on tympanic membrane compliance
- Use a personalized REOIG measure for each earpiece type based on estimated ear canal volume (which is also a result of tympanometry)
- Measure the actual REOIG for multiple earpiece types in the individual patient

Hearing aid fitting system:

In an aspect of the present application, a hearing aid fitting system is provided.

The hearing aid fitting system may comprise a database for storing ACT data of at least one ear of a hearing aid user.

The hearing aid fitting system may comprise a database for storing an audiogram of the hearing aid user.

The hearing aid fitting system may comprise a database for storing an age parameter of the hearing aid user.

The hearing aid fitting system may comprise a hearing aid fitting device.

The hearing aid fitting device may be configured to receive said stored ACT data from the database.

The hearing aid fitting device may be configured to receive said audiogram from the database.

The hearing aid fitting device may be configured to receive said age parameter from the database.

The hearing aid fitting device may be configured to determine signal processing parameter settings for a hearing aid according to a prescription protocol in dependence of said ACT value, audiogram, and age parameter.

The hearing aid may be suitable for use by the hearing aid user.

In dependence of may refer to that the prescription protocol takes into account the ACT value, audiogram, and age parameter of the hearing aid user, when determining a prescription level to be assigned to the hearing aid user.

The hearing aid fitting device may be configured to transmit said signal processing parameter settings to the hearing aid for setting signal processing parameters of the hearing aid.

The hearing aid fitting device may comprise a processor configured to determine said signal processing parameter settings for the hearing aid.

Fitting software (a computer program) for determining said signal processing parameter settings for the hearing aid according to the prescription protocol may be stored on the hearing aid fitting device (e.g., on a memory) and be executed by the processor.

The hearing aid fitting system may further comprise an analysis unit for determining the regression model.

The linear regression model may be estimated by the analysis unit based on pre-determined ACT data, audiograms, and/or age parameters from a multitude of users. The linear regression model may be suitable as prescription protocol for defining the multitude of prescription levels.

For example, the audiograms may be PTA as a four-frequency average, PTA4, across two ears of each of the users.

In other words, coefficients of the linear regression model may be estimated/determined by the analysis unit. For example, the coefficients may comprise an ACT value specific coefficient, a PTA value specific coefficient, and/or an age parameter specific coefficient. Further, signal processing parameters corresponding to the regression model may be estimated/determined by the analysis unit. In other words, the analysis unit may be configured to define a multitude of prescription levels, where each prescription level comprises pre-set signal processing parameters.

The hearing aid fitting device may receive the linear regression model as determined by the analysis unit. The received linear regression model may comprise said estimated/determined coefficients.

The hearing aid fitting device may be configured to determine the prescription level of a given hearing aid user by use of the linear regression model, with input of the ACT value, the audiogram (e.g., the PTA value) and the age parameter determined from of the hearing aid user.

A hearing aid:

The hearing aid may be constituted by or comprising an air-conduction type hearing aid.

The hearing aid may be constituted by or comprising a bone-conduction type hearing aid.

The hearing aid may be constituted by or comprising any combination of the air-conduction type hearing aid and the bone-conduction type hearing aid.

The hearing aid may be adapted to provide a frequency dependent gain and/or a level dependent compression and/or a transposition (with or without frequency compression) of one or more frequency ranges to one or more other frequency ranges, e.g. to compensate for a hearing impairment of a user.

The hearing aid may comprise an output unit for providing a stimulus perceived by the user as an acoustic signal based on a processed electric signal. The output unit may a vibrator of a bone conducting hearing aid. The output unit may comprise an output transducer. The output transducer may comprise a receiver (loudspeaker) for providing the stimulus as an acoustic signal to the user (e.g. in an acoustic (air conduction based) hearing aid). The output transducer may comprise a vibrator for providing the stimulus as mechanical vibration of a skull bone to the user (e.g. in a bone-attached or bone-anchored hearing aid). The output unit may (additionally or alternatively) comprise a (e.g. wireless) transmitter for transmitting sound picked up-by the hearing aid to another device, e.g. a far-end communication partner (e.g. via a network, e.g. in a telephone mode of operation, or in a headset configuration).

The hearing aid may comprise an input unit for providing an electric input signal representing sound. The input unit may comprise an input transducer, e.g. a microphone, for converting an input sound to an electric input signal. The input unit may comprise a wireless receiver for receiving a wireless signal comprising or representing sound and for providing an electric input signal representing said sound.

The wireless receiver and/or transmitter may e.g. be configured to receive and/or transmit an electromagnetic signal in the radio frequency range (3 kHz to 300 GHz). The wireless receiver and/or transmitter may e.g. be configured to receive and/or transmit an electromagnetic signal in a frequency range of light (e.g. infrared light 300 GHz to 430 THz, or visible light, e.g. 430 THz to 770 THz).

The hearing aid may comprise a directional microphone system adapted to spatially filter sounds from the environment, and thereby enhance a target acoustic source among a multitude of acoustic sources in the local environment of the user wearing the hearing aid. The directional system may be adapted to detect (such as adaptively detect) from which direction a particular part of the microphone signal originates. This can be achieved in various different ways as e.g. described in the prior art. In hearing aids, a microphone array beamformer is often used for spatially attenuating background noise sources. The beamformer may comprise a linear constraint minimum variance (LCMV) beamformer. Many beamformer variants can be found in literature. The minimum variance distortionless response (MVDR) beamformer is widely used in microphone array signal processing. Ideally the MVDR beamformer keeps the signals from the target direction (also referred to as the look direction) unchanged, while attenuating sound signals from other directions maximally. The generalized sidelobe canceller (GSC) structure is an equivalent representation of the MVDR beamformer offering computational and numerical advantages over a direct implementation in its original form. In other words, the one or more prescription levels may each comprise a beamforming and/or noise reduction setting based on the above directional microphone system.

Most sound signal sources (except the user's own voice) are located far away from the user compared to dimensions of the hearing aid, e.g. a distance $d_{mic}$ between two microphones of a directional system. A typical microphone distance in a hearing aid is of the order 10 mm. A *minimum* distance of a sound source of interest to the user (e.g. sound from the user's mouth or sound from an audio delivery device) is of the order of 0.1 m (> 10 $d_{mic}$). For such minimum distances, the hearing aid (microphones) would be in the acoustic near-field of the sound source and a difference in level of the sound signals impinging on respective microphones may be significant. A *typical* distance for a communication partner is more than 1 m (>100 $d_{mic}$). The hearing aid (microphones) would be in the acoustic far-field of the sound source and a difference in level of the sound signals impinging on respective microphones is insignificant. The difference in *time of arrival* of sound impinging in the direction of the microphone axis (e.g. the front or back of a normal hearing aid) is $\Delta T = d_{mic}/v_{sound} = 0.01/343 [s] = 29 \mu s$, where $v_{sound}$ is the speed of sound in air at 20°C (343 m/s).

The hearing aid may comprise antenna and transceiver circuitry allowing a wireless link to an entertainment device (e.g. a TV-set), a communication device (e.g. a telephone), a wireless microphone, a separate (external) processing device, or another hearing aid, etc. The hearing aid may thus be configured to wirelessly receive a direct electric input signal from another device. Likewise, the hearing aid may be configured to wirelessly transmit a direct electric output signal to another device. The direct electric input or output signal may represent or comprise an audio signal and/or a control signal and/or an information signal.

For example, the hearing aid may be configured to be in communication with the hearing aid fitting device via the antenna and transceiver circuitry so as to receive said signal processing parameter settings. Similarly, the hearing aid fitting device may comprise antenna and transceiver circuitry, thereby being configured to communicate with the database of the hearing aid fitting system. Alternatively, or additionally, the hearing aid fitting device, the database, and/or the hearing aid may be configured to communicate with each other via a wired connection.

In general, a wireless link established by antenna and transceiver circuitry of the hearing aid can be of any type. The wireless link may be a link based on near-field communication, e.g. an inductive link based on an inductive coupling between antenna coils of transmitter and receiver parts. The wireless link may be based on far-field, electromagnetic radiation. Preferably, frequencies used to establish a communication link between the hearing aid and the other device is below 70 GHz, e.g. located in a range from 50 MHz to 70 GHz, e.g. above 300 MHz, e.g. in an ISM range above 300 MHz, e.g. in the 900 MHz range or in the 2.4 GHz range or in the 5.8 GHz range or in the 60 GHz range (ISM=Industrial, Scientific and Medical, such standardized ranges being e.g. defined by the International Telecommunication Union, ITU). The wireless link may be based on a standardized or proprietary technology. The wireless link may be based on Bluetooth technology (e.g. Bluetooth Low-Energy technology, e.g. LE audio), or Ultra WideBand (UWB) technology.

The hearing aid may be constituted by or form part of a portable (i.e. configured to be wearable) device, e.g. a device comprising a local energy source, e.g. a battery, e.g. a rechargeable battery. The hearing aid may e.g. be a low weight, easily wearable, device, e.g. having a total weight less than 100 g, such as less than 20 g, such as less than 5 g.

The hearing aid may comprise a 'forward' (or 'signal') path for processing an audio signal between an input and an output of the hearing aid. A signal processor may be located in the forward path. The signal processor may be adapted to provide a frequency dependent gain according to a user's particular needs (e.g. hearing impairment). The hearing aid may comprise an 'analysis' path comprising functional components for analyzing signals and/or controlling processing of the forward path. Some or all signal processing of the analysis path and/or the forward path may be conducted in the frequency domain, in which case the hearing aid comprises appropriate analysis and synthesis filter banks. Some or all signal processing of the analysis path and/or the forward path may be conducted in the time domain.

An analogue electric signal representing an acoustic signal may be converted to a digital audio signal in an analogue-to-digital (AD) conversion process, where the analogue signal is sampled with a predefined sampling frequency or rate $f_s$, $f_s$ being e.g. in the range from 8 kHz to 48 kHz (adapted to the particular needs of the application) to provide digital samples $x_n$ (or x[n]) at discrete points in time $t_n$ (or n), each audio sample representing the value of the acoustic signal at $t_n$ by a predefined number $N_b$ of bits, $N_b$ being e.g. in the range from 1 to 48 bits, e.g. 24 bits. Each audio sample is hence quantized using $N_b$ bits (resulting in $2^{Nb}$ different possible values of the audio sample). A digital sample x has a length in time of $1/f_s$, e.g. 50 $\mu s$, for $f_s$ = 20 kHz. A number of audio samples may be arranged in a time frame. A time frame may comprise 64 or 128 audio data samples. Other frame lengths may be used depending on the practical application.

The hearing aid may comprise an analogue-to-digital (AD) converter to digitize an analogue input (e.g. from an input transducer, such as a microphone) with a predefined sampling rate, e.g. 20 kHz. The hearing aids may comprise a digital-to-analogue (DA) converter to convert a digital signal to an analogue output signal, e.g. for being presented to a user via an output transducer.

The hearing aid, e.g. the input unit, and or the antenna and transceiver circuitry may comprise a transform unit for converting a time domain signal to a signal in the transform domain (e.g. frequency domain or Laplace domain, Z transform, wavelet transform, etc.). The transform unit may be constituted by or comprise a TF-conversion unit for providing a time-frequency representation of an input signal. The time-frequency representation may comprise an array or map of corresponding complex or real values of the signal in question in a particular time and frequency range. The TF conversion unit may comprise a filter bank for filtering a (time varying) input signal and providing a number of (time varying) output signals each comprising a distinct frequency range of the input signal. The TF conversion unit may comprise a Fourier transformation unit (e.g. a Discrete Fourier Transform (DFT) algorithm, or a Short Time Fourier Transform (STFT) algorithm, or similar) for converting a time variant input signal to a (time variant) signal in the (time-)frequency domain. The frequency range considered by the hearing aid from a minimum frequency $f_{min}$ to a maximum frequency $f_{max}$ may comprise a part of the typical human audible frequency range from 20 Hz to 20 kHz, e.g. a part of the range from 20 Hz to 12 kHz. Typically, a sample rate $f_s$ is larger than or equal to twice the maximum frequency $f_{max}$, $f_s \geq 2f_{max}$. A signal of the forward and/or analysis path of the hearing aid may be split into a number $NI$ of frequency bands (e.g. of uniform width), where $NI$ is e.g. larger than 5, such as larger than 10, such as larger than 50, such as larger than 100, such as larger than 500, at least some of which are processed individually. The hearing aid may be adapted to process a signal of the forward and/or analysis path in a number $NP$ of different frequency channels ($NP \leq NI$). The frequency channels may be uniform or non-uniform in width (e.g. increasing in width with frequency), overlapping or non-overlapping.

The hearing aid may be configured to operate in different modes, e.g. a normal mode and one or more specific modes, e.g. selectable by a user, or automatically selectable. A mode of operation may be optimized to a specific acoustic situation or environment, e.g. a communication mode, such as a telephone mode. A mode of operation may include a lowpower mode, where functionality of the hearing aid is reduced (e.g. to save power), e.g. to disable wireless communication, and/or to disable specific features of the hearing aid.

For example, the mode of operation may refer to and/or be set by said one or more prescription levels.

The hearing aid may comprise a number of detectors configured to provide status signals relating to a current physical environment of the hearing aid (e.g. the current acoustic environment), and/or to a current state of the user wearing the hearing aid, and/or to a current state or mode of operation of the hearing aid. Alternatively, or additionally, one or more detectors may form part of an *external* device in communication (e.g. wirelessly) with the hearing aid. An external device may e.g. comprise another hearing aid, a remote control, and audio delivery device, a telephone (e.g. a smartphone), an external sensor, etc.

One or more of the number of detectors may operate on the full band signal (time domain). One or more of the number of detectors may operate on band split signals ((time-) frequency domain), e.g. in a limited number of frequency bands.

The number of detectors may comprise a level detector for estimating a current level of a signal of the forward path. The detector may be configured to decide whether the current level of a signal of the forward path is above or below a given (L-)threshold value. The level detector operates on the full band signal (time domain). The level detector operates on band split signals ((time-) frequency domain).

The hearing aid may comprise a voice activity detector (VAD) for estimating whether or not (or with what probability) an input signal comprises a voice signal (at a given point in time). A voice signal may in the present context be taken to include a speech signal from a human being. It may also include other forms of utterances generated by the human speech system (e.g. singing). The voice activity detector unit may be adapted to classify a current acoustic environment of the user as a VOICE or NO-VOICE environment. This has the advantage that time segments of the electric microphone signal comprising human utterances (e.g. speech) in the user's environment can be identified, and thus separated from time segments only (or mainly) comprising other sound sources (e.g. artificially generated noise). The voice activity detector may be adapted to detect as a VOICE also the user's own voice. Alternatively, the voice activity detector may be adapted to exclude a user's own voice from the detection of a VOICE.

The hearing aid may comprise an own voice detector for estimating whether or not (or with what probability) a given input sound (e.g. a voice, e.g. speech) originates from the voice of the user of the system. A microphone system of the hearing aid may be adapted to be able to differentiate between a user's own voice and another person's voice and possibly from NON-voice sounds.

The number of detectors may comprise a movement detector, e.g. an acceleration sensor. The movement detector may be configured to detect movement of the user's facial muscles and/or bones, e.g. due to speech or chewing (e.g. jaw movement) and to provide a detector signal indicative thereof.

The hearing aid may comprise a classification unit configured to classify the current situation based on input signals from (at least some of) the detectors, and possibly other inputs as well. In the present context 'a current situation' may be taken to be defined by one or more of

a) the physical environment (e.g. including the current electromagnetic environment, e.g. the occurrence of electromagnetic signals (e.g. comprising audio and/or control signals) intended or not intended for reception by the hearing aid, or other properties of the current environment than acoustic);
b) the current acoustic situation (input level, feedback, etc.);

c) the current mode or state of the user (movement, temperature, cognitive load, etc.);

d) the current mode or state of the hearing aid (program selected, time elapsed since last user interaction, etc.) and/or of another device in communication with the hearing aid.

The classification unit may be based on or comprise a neural network, e.g. a recurrent neural network, e.g. a trained neural network.

The hearing aid may comprise an acoustic (and/or mechanical) feedback control (e.g. suppression) or echo-cancelling system. Adaptive feedback cancellation has the ability to track feedback path changes over time. It is typically based on a linear time invariant filter to estimate the feedback path, but its filter weights are updated over time. The filter update may be calculated using stochastic gradient algorithms, including some form of the Least Mean Square (LMS) or the Normalized LMS (NLMS) algorithms. They both have the property to minimize the error signal in the mean square sense with the NLMS additionally normalizing the filter update with respect to the squared Euclidean norm of some reference signal.

The hearing aid may further comprise other relevant functionality for the application in question, e.g. compression, noise reduction, etc. The other relevant functionality may be part of said one or more prescription levels.

The hearing aid may comprise a hearing instrument, e.g. a hearing instrument adapted for being located at the ear or fully or partially in the ear canal of a user, e.g. a headset, an earphone, an ear protection device or a combination thereof. A hearing system may comprise a speakerphone (comprising a number of input transducers (e.g. a microphone array) and a number of output transducers, e.g. one or more loudspeakers, and one or more audio (and possibly video) transmitters e.g. for use in an audio conference situation), e.g. comprising a beamformer filtering unit, e.g. providing multiple beamforming capabilities.

It is intended that some or all of the structural features of the hearing aid and/or the hearing aid fitting system described above, in the 'detailed description of embodiments' or in the claims can be combined with embodiments of the method, when appropriately substituted by a corresponding process and vice versa. Embodiments of the method have the same advantages as the corresponding aids/devices/systems.

Use:

In an aspect, use of a hearing aid and a hearing aid fitting system as described above, in the 'detailed description of embodiments' and in the claims, is moreover provided. Use may be provided in a system comprising one or more hearing aids (e.g. hearing instruments), headsets, earphones, active ear protection systems, etc., e.g. in handsfree telephone systems, teleconferencing systems (e.g. including a speakerphone), public address systems, karaoke systems, classroom amplification systems, etc.

A computer readable medium or data carrier:

In an aspect, a tangible computer-readable medium (a data carrier) storing a computer program comprising program code means (instructions) for causing a data processing system (a computer) to perform (carry out) at least some (such as a majority or all) of the (steps of the) method described above, in the 'detailed description of embodiments' and in the claims, when said computer program is executed on the data processing system is furthermore provided by the present application.

By way of example, and not limitation, such computer-readable media can comprise RAM, ROM, EEPROM, CD-ROM or other optical disk storage, magnetic disk storage or other magnetic storage devices, or any other medium that can be used to carry or store desired program code in the form of instructions or data structures and that can be accessed by a computer. Disk and disc, as used herein, includes compact disc (CD), laser disc, optical disc, digital versatile disc (DVD), floppy disk and Blu-ray disc where disks usually reproduce data magnetically, while discs reproduce data optically with lasers. Other storage media include storage in DNA (e.g. in synthesized DNA strands). Combinations of the above should also be included within the scope of computer-readable media. In addition to being stored on a tangible medium, the computer program can also be transmitted via a transmission medium such as a wired or wireless link or a network, e.g. the Internet, and loaded into a data processing system for being executed at a location different from that of the tangible medium.

A computer program:

A computer program (product) comprising instructions which, when the program is executed by a computer (e.g., as part of the hearing aid fitting device or hearing aid), cause the computer to carry out (steps of) the method described above, in the 'detailed description of embodiments' and in the claims is furthermore provided by the present application.

A data processing system:

In an aspect, a data processing system comprising a processor and program code means for causing the processor to perform at least some (such as a majority or all) of the steps of the method described above, in the 'detailed description of embodiments' and in the claims is furthermore provided by the present application.

A hearing system:

In a further aspect, a hearing system comprising a hearing aid as described above, in the 'detailed description of embodiments', and in the claims, AND an auxiliary device is moreover provided.

The hearing system may be adapted to establish a communication link between the hearing aid and the auxiliary device to provide that information (e.g. control and status signals, possibly audio signals) can be exchanged or forwarded from one to the other.

The auxiliary device may be constituted by or comprise a remote control, a smartphone, or other portable or wearable electronic device, such as a smartwatch or the like.

The auxiliary device may be constituted by or comprise a remote control for controlling functionality and operation of the hearing aid(s). The function of a remote control may be implemented in a smartphone, the smartphone possibly running an APP allowing to control the functionality of the audio processing device via the smartphone (the hearing aid(s) comprising an appropriate wireless interface to the smartphone, e.g. based on Bluetooth or some other standardized or proprietary scheme).

The auxiliary device may be constituted by or comprise an audio gateway device adapted for receiving a multitude of audio signals (e.g. from an entertainment device, e.g. a TV or a music player, a telephone apparatus, e.g. a mobile telephone or a computer, e.g. a PC, a wireless microphone, etc.) and adapted for selecting and/or combining an appropriate one of the received audio signals (or combination of signals) for transmission to the hearing aid.

The auxiliary device may be constituted by or comprise another hearing aid. The hearing system may comprise two hearing aids adapted to implement a binaural hearing system, e.g. a binaural hearing aid system.

For example, the signal processing parameter settings for a hearing aid for use by the hearing aid user according to a prescription protocol may be designed to be implemented in a binaural hearing system.

An APP:

In a further aspect, a non-transitory application, termed an APP, is furthermore provided by the present disclosure. The APP comprises executable instructions configured to be executed on an auxiliary device to implement a user interface for a hearing aid or a hearing system described above in the 'detailed description of embodiments', and in the claims. The APP may be configured to run on cellular phone, e.g. a smartphone, or on another portable device allowing communication with said hearing aid or said hearing system.

Definitions:

In the present context, a hearing aid, e.g. a hearing instrument, refers to a device, which is adapted to improve, augment and/or protect the hearing capability of a user by receiving acoustic signals from the user's surroundings, generating corresponding audio signals, possibly modifying the audio signals and providing the possibly modified audio signals as audible signals to at least one of the user's ears. Such audible signals may e.g. be provided in the form of acoustic signals radiated into the user's outer ears and/or acoustic signals transferred as mechanical vibrations to the user's inner ears through the bone structure of the user's head and/or through parts of the middle ear.

The hearing aid may be configured to be worn in any known way, e.g. as a unit arranged behind the ear with a tube leading radiated acoustic signals into the ear canal or with an output transducer, e.g. a loudspeaker, arranged close to or in the ear canal, as a unit entirely or partly arranged in the pinna and/or in the ear canal, as a unit, e.g. a vibrator, attached to a fixture implanted into the skull bone, etc. The hearing aid may comprise a single unit or several units communicating (e.g. acoustically, electrically or optically) with each other. The loudspeaker may be arranged in a housing together with other components of the hearing aid, or may be an external unit in itself (possibly in combination with a flexible guiding element, e.g. a domelike element).

A hearing aid may be adapted to a particular user's needs, e.g. a hearing impairment. A configurable signal processing circuit of the hearing aid may be adapted to apply a frequency and level dependent compressive amplification of an input signal. A customized frequency and level dependent gain (amplification or compression) may be determined in a fitting process by a fitting system based on a user's hearing data, e.g. an audiogram, using a fitting rationale (e.g. adapted to speech). The frequency and level dependent gain may e.g. be embodied in processing parameters, e.g. uploaded to the hearing aid via an interface to a programming device (fitting system), and used by a processing algorithm executed by the configurable signal processing circuit of the hearing aid.

A 'hearing system' refers to a system comprising one or two hearing aids, and a 'binaural hearing system' refers to a system comprising two hearing aids and being adapted to cooperatively provide audible signals to both of the user's ears. Hearing systems or binaural hearing systems may further comprise one or more auxiliary devices', which communicate with the hearing aid(s) and affect and/or benefit from the function of the hearing aid(s). Such auxiliary devices may include at least one of a remote control, a remote microphone, an audio gateway device, an entertainment device, e.g. a music player, a wireless communication device, e.g. a mobile phone (such as a smartphone) or a tablet or another device, e.g. comprising a graphical interface. Hearing aids, hearing systems or binaural hearing systems may e.g. be used for compensating for a hearing-impaired person's loss of hearing capability, augmenting or protecting a normal-hearing person's hearing capability and/or conveying electronic audio signals to a person. Hearing aids or hearing systems may e.g. form part of or interact with public-address systems, active ear protection systems, handsfree telephone systems, car audio systems, entertainment (e.g. TV, music playing or karaoke) systems, teleconferencing systems, classroom amplification systems, etc.

The invention is set out in the appended set of claims.

BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:

FIG. 1 shows an exemplary hearing aid fitting system according to the present application.

FIG. 2 shows an exemplary method of setting signal processing parameters of a hearing aid according to the present application.

FIG. 3 shows an exemplary prescription protocol and corresponding prescription levels.

The figures are schematic and simplified for clarity, and they just show details which are essential to the understanding of the disclosure, while other details are left out. Throughout, the same reference signs are used for identical or corresponding parts.

Further scope of applicability of the present disclosure will become apparent from the detailed description given hereinafter. However, it should be understood that the detailed description and specific examples, while indicating preferred embodiments of the disclosure, are given by way of illustration only. Other embodiments may become apparent to those skilled in the art from the following detailed description.

DETAILED DESCRIPTION OF EMBODIMENTS

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the apparatus and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

The electronic hardware may include micro-electronic-mechanical systems (MEMS), integrated circuits (e.g. application specific), microprocessors, microcontrollers, digital signal processors (DSPs), field programmable gate arrays (FPGAs), programmable logic devices (PLDs), gated logic, discrete hardware circuits, printed circuit boards (PCB) (e.g. flexible PCBs), and other suitable hardware configured to perform the various functionality described throughout this disclosure, e.g. sensors, e.g. for sensing and/or registering physical properties of the environment, the device, the user, etc. Computer program shall be construed broadly to mean instructions, instruction sets, code, code segments, program code, programs, subprograms, software modules, applications, software applications, software packages, routines, subroutines, objects, executables, threads of execution, procedures, functions, etc., whether referred to as software, firmware, middleware, microcode, hardware description language, or otherwise.

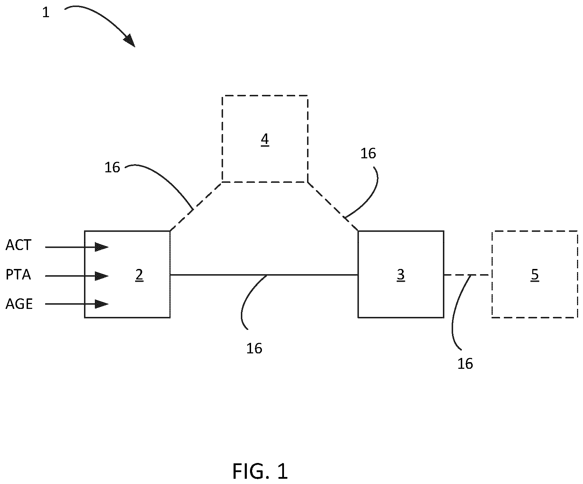

FIG. 1 shows an exemplary hearing aid fitting system according to the present application.

The hearing aid fitting system 1 shown in FIG. 1, may comprise a database 2 and a hearing aid fitting device 3.

The database 2 may may be configured to store ACT data (e.g., ACT value) measured of at least one ear of a hearing aid user (e.g., a binaural measurement of ACT data may be stored), an audiogram of the hearing aid user, and an age parameter AGE of the hearing aid user. As indicated, the audiogram may be pre-processed to form a PTA value either before being stored or in a following step.

The database 2 may additionally store pre-determined ACT data, audiograms, and/or age parameters determined/measured from a multitude of users.

The database 2 may additionally store a pre-determined linear regression model suitable for use by the hearing aid fitting device 3, when determining prescription level of a hearing aid for a given hearing aid user.

The hearing aid fitting system 1 may comprise an analysis unit 4. The analysis unit 4 may be configured to receive said pre-determined ACT data, audiograms, and/or age parameters determined/measured from a multitude of users, from the database 2 (or from an additional database not shown).

The analysis unit 4 may determining the regression model. Accordingly, the linear regression model may be estimated by the analysis unit 4 based on said pre-determined ACT data, audiograms (e.g., PTA values), and/or age parameters from a multitude of users. The linear regression model may be suitable as prescription protocol for defining the multitude of prescription levels. In other words, coefficients of the linear regression model may be estimated/determined by the analysis unit 4. For example, the coefficients may comprise an ACT value specific coefficient, a PTA value specific coefficient, and/or an age parameter specific coefficient. Further, signal processing parameters corresponding to the regression model may be estimated/determined by the analysis unit 4. In other words, the analysis unit 4 may be configured to define a multitude of prescription levels, where each prescription level comprises pre-set signal processing parameters.

The hearing aid fitting device 3 may be configured to receive the linear regression model, or at least the coefficients of the linear regression model, from the analysis unit 4. Alternatively, the linear regression model or said coefficients may be stored at the database 2, and be send to the hearing aid fitting device 3.

The hearing aid fitting device may further receive said ACT data (e.g., ACT value) measured of at least one ear of a hearing aid user, said audiogram (e.g., PTA value) of the hearing aid user, and an age parameter AGE of the hearing aid user from the database 2. Thereby, the hearing aid fitting device 3 may determine the prescription level of the given hearing aid user by use of the linear regression model, with input of the ACT value, the PTA value, and the age parameter determined from of the hearing aid user.

The determined prescription level of the given hearing aid user may be used to fit the hearing aid 5 of the hearing aid user.

The database 2, the hearing aid fitting device 3, the analysis unit 4, and the hearing aid 5 may be configured to be in communication with each other as indicated by the solid and dotted lines 16 either via a wired or wireless connection.

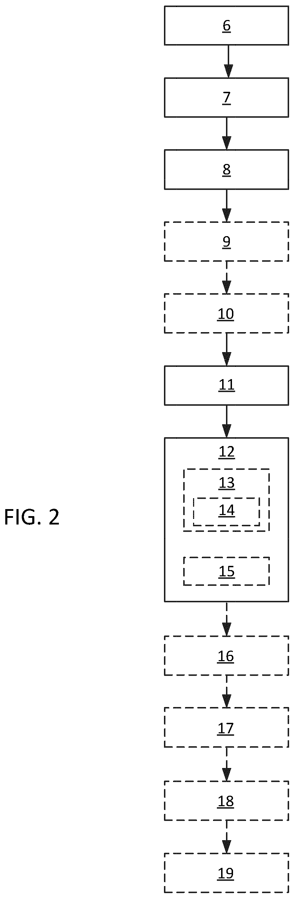

FIG. 2 shows an exemplary method of setting signal processing parameters of a hearing aid according to the present application.

As shown in FIG. 2, the method may comprise a step 6 of determining ACT data, such an ACT value, of at least one ear (e.g., binaurally) of a hearing aid user.

The method may further comprise a step 7 of determining an audiogram (e.g., a PTA value) of the hearing aid user.

The method may further comprise a step 8 of determining an age parameter of the hearing aid user.

The method may further comprise a step 9 of pre-processing said ACT data and/or said audiogram to reduce dimensionality.

A linear regression model may be determined 10 (or alternatively received if already determined), based on pre-determined ACT data, audiograms (e.g., PTA values), and/or age parameters from a multitude of users.

Based on the determined linear regression model and the hearing aid user specific ACT value, PTA value, and age parameter, signal processing parameter settings for a hearing aid for use by the hearing aid user according to a prescription protocol may be determined 11.

The method may further comprise setting 12 signal processing parameters of the hearing aid. The signal processing parameters may be set according to a prescription protocol. In other words, a multitude of prescription levels may be defined 13, where each prescription level may comprise pre-set signal processing parameters.

Defining 14 a multitude of prescription levels may comprise determining a linear regression model as prescription protocol for defining said multitude of prescription levels in dependence of said ACT value, audiogram, and age parameter.

The step of setting 12 signal processing parameters of the hearing aid may further comprise transmitting 15 said determined signal processing parameter settings to the hearing aid.

The method may further comprise estimating 16 an acoustic coupling. The acoustic coupling may be estimated in terms of REOIG required to obtain the determined 12 signal processing parameter settings (i.e., the SNR enhancement).

The method may further comprise identifying 17 earpiece type and vent size. The earpiece type and vent size may be the one that provides the required acoustic coupling estimated above.

The method may further comprise providing 18 an upper limit for the acoustic coupling.

The method may further comprise prescribing 19 earpiece and vent size based on the step of identifying 17 earpiece type and vent size and the step of providing 18 an upper limit for the acoustic coupling.

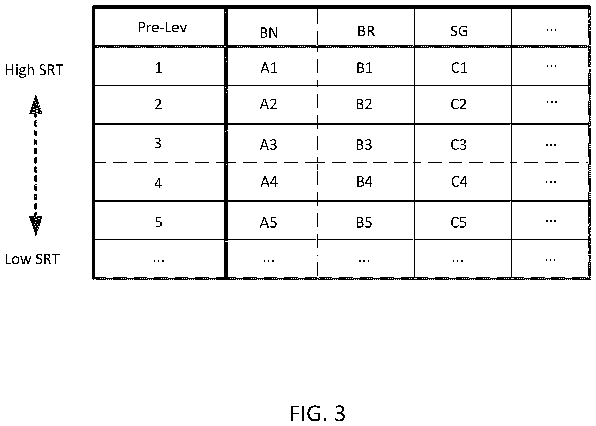

FIG. 3 shows an exemplary prescription protocol and corresponding prescription levels.

In FIG. 3, an exemplary table illustrating how the prescription level may be determined for a specific hearing aid user, and how the corresponding signal processing parameters may be determined.

From the linear regression model, an SRT may be estimated for the hearing aid user, e.g., by use of the equation (see also above):

$$SRT = k_{ACT} \times ACT + k_{PTA} \times PTA + k_{AGE} \times Age + C$$

A multitude of prescription levels Pre-Lev may be defined, starting with a high (preset) SRT value and going towards a low SRT value. The prescription levels Pre-Lev are shown to be termed 1 to 5, but other naming is foreseen.

Each of the prescription levels may comprise a range of SRT levels.

The prescription levels may be separated by SRT thresholds, so that each estimated SRT value of the hearing aid user corresponds to one of the prescription levels Pre-Lev.

For each prescription level Pre-Lev, one or more signal processing parameter settings may be defined. The amount of aid (e.g., help-in-noise) may vary from level to level Pre-Lev. For example, in prescription level Pre-Lev 1 a higher aid is provided than in prescription level Pre-Lev 2.

In FIG. 3, it is shown that said settings may comprise beamforming and/or noise reduction settings BN, high frequency gain (brightness) settings BR, and listening distance (soft gain) settings SG. However, it is foreseen that even more signal processing parameter settings may be set, such as for example transient noise reduction.

It is intended that the structural features of the devices described above, either in the detailed description and/or in the claims, may be combined with steps of the method, when appropriately substituted by a corresponding process.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element, but an intervening element may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method are not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art.

The claims are not intended to be limited to the aspects shown herein but are to be accorded the full scope consistent with the language of the claims, wherein reference to an element in the singular is not intended to mean "one and only one" unless specifically so stated, but rather "one or more." Unless specifically stated otherwise, the term "some" refers to one or more.

References:

[1] Zaar, J., Simonsen, L. B., Behrens, T., Dau, T., & Laugesen, S. (2019). Investigating the relationship between spectro-temporal modulation detection, aided speech perception, and directional noise reduction preference in hearing-impaired listeners. Proceedings of the International Symposium on Auditory and Audiological Research, 7, 181-188.

[2] Cubick, J., Caporali, S., Lelic, D., Catic, J., Vikær Damsgaard, A., Rose, S., Ives, T. Schmidt, E. (2022). The Acoustics of Instant Ear Tips and Their Implications for Hearing-Aid Fitting. Ear & Hearing, 43(6), 1771-1782.

[3] Carle, R., Laugesen, S., Nielsen, C. (2002). Observations on the Relations among Occlusion Effect, Compliance, and Vent Size. J. Am. Acad. Audiol., 13(1), 25-37.

**Claims**

1. Method of setting signal processing parameters of a hearing aid, the method comprising:

   - determining Audible Contrast Threshold (ACT) data of at least one ear of a hearing aid user,
   - determining an audiogram of the hearing aid user,
   - determining an age parameter of the hearing aid user,
   - determining signal processing parameter settings for a hearing aid for use by the hearing aid user according to a prescription protocol in dependence of said ACT data, audiogram, and age parameter,
   - setting signal processing parameters of the hearing aid.

2. Method according to claim 1, wherein said ACT data comprises an ACT value that is a normalized contrast level (nCL) value where 0 dB nCL corresponds to a median performance of a test group of normal hearing test subjects, and where 16 dB nCL corresponds to the physically maximal degree of spectro-temporal modulation, that is, 0 dB Full Scale (FS).

3. Method according to any one of the preceding claims, wherein the step of determining ACT data comprises:

   - estimating ACT data of at least one of the ears of the hearing aid user in an ACT test, or
   - receiving estimated ACT data of the hearing aid user from a database.

4. Method according to any one of the preceding claims, wherein the step of determining an audiogram of the hearing aid user comprises:

   - estimating one or more Hearing Threshold Level (HTL) values from a measured audiogram of the hearing aid user, or
   - receiving a measured audiogram or estimated HTL values from a database.

5. Method according to any one of the preceding claims, wherein the step of determining an age parameter comprises:

   - inputting an age parameter of the hearing aid user, or
   - receiving an age parameter of the hearing aid user from a database.

6. Method according to any one of the preceding claims, wherein the step of determining ACT data and/or an audiogram of the hearing aid user comprises:

   - pre-processing said ACT data and/or said audiogram to reduce dimensionality.

7. Method according to claim 6, wherein the step of pre-processing said audiogram comprises:

   - estimating HTL values at a range of audiometric frequencies, and
   - estimating a four-frequency average value, e.g. a Pure-Tone Average (PTA) value.

8. Method according to any one of the preceding claims, wherein the step of setting signal processing parameters of the hearing aid according to a prescription protocol comprises:

   - defining a multitude of prescription levels, where each prescription level comprises pre-set signal processing parameters.

9. Method according to claim 8, wherein defining a multitude of prescription levels comprises:

   - determining a linear regression model as prescription protocol for defining said multitude of prescription levels in dependence of said ACT value, audiogram, and age parameter.

10. Method according to any one of claims 8-9, wherein said multitude of prescription levels are separated by speech reception threshold (SRT) thresholds, and where said linear regression model estimates an SRT value of the hearing aid user.

11. Method according to any one of claims 9-10, wherein said linear regression model is estimated based on pre-determined ACT data, audiograms, age parameters, and signal processing parameters.

12. Method according to any one of the preceding claims, wherein the multitude of prescription levels each comprises:

  - a beamforming and/or noise reduction setting,
  - a high frequency gain (brightness) setting,
  - a listening distance (soft gain) setting, and/or
  - a transient noise reduction setting.

13. Method according to any one of the preceding claims, wherein the step of determining signal processing parameter settings for a hearing aid for use by the hearing aid user according to a prescription protocol in dependence of said ACT value, audiogram, and age parameter comprises:

  - determining a value representative of one of said prescription levels.

14. Method according to any one of the preceding claims, wherein the method further comprises to determine an acoustic coupling by estimating a real-ear occluded insertion gain (REOIG) required to obtain the determined signal processing parameter settings of the hearing aid.

15. Hearing aid fitting system comprising:

  - a database for storing ACT data of at least one ear of a hearing aid user, an audiogram of the hearing aid user, and an age parameter of the hearing aid user,
  - a hearing aid fitting device configured to receive said stored ACT data, audiogram, and age parameter from the database, where the hearing aid fitting device is configured to determine signal processing parameter settings for a hearing aid for use by the hearing aid user according to a prescription protocol in dependence of said ACT value, audiogram, and age parameter, and where the hearing aid fitting device is configured to transmit said signal processing parameter settings to the hearing aid for setting signal processing parameters of the hearing aid.

16. Hearing aid fitting system according to any one of claims 15, wherein the hearing aid being constituted by or comprising an air-conduction type hearing aid, a bone-conduction type hearing aid, or any combination thereof.

17. A data processing system comprising a processor and program code means for causing the processor to perform at least some of the steps of the method of any one of claims 1-14.

18. A computer program comprising instructions which, when the program is executed by a computer, cause the computer to carry out the method of any one of claims 1-14.

FIG. 1

FIG. 2

| Pre-Lev | BN | BR | SG | ... |
|---------|-----|-----|-----|-----|
| 1 | A1 | B1 | C1 | ... |
| 2 | A2 | B2 | C2 | ... |
| 3 | A3 | B3 | C3 | ... |
| 4 | A4 | B4 | C4 | ... |
| 5 | A5 | B5 | C5 | ... |
| ... | ... | ... | ... | ... |

High SRT

Low SRT

FIG. 3

Europäisches
Patentamt

European
Patent Office

Office européen
des brevets

# EUROPEAN SEARCH REPORT

**Application Number**

EP 25 15 4843

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/037356 A1 (PONTOPPIDAN NIELS HENRIK [DK] ET AL) 9 February 2023 (2023-02-09) | 1,3-8, 12-18 | INV. H04R25/00 |
| Y | * paragraph [0032] - paragraph [0208]; figures 1A, 7A * | 1-18 | |
| X | PEDERSEN CARL CHRISTIAN ET AL: "Comparison of hearing aid fitting effectiveness with audiograms from either user-operated or traditional audiometry in a clinical setting: a study protocol for a blinded non-inferiority randomised controlled trial", BMJ OPEN, vol. 13, no. 3, 1 March 2023 (2023-03-01), page e065777, XP093180907, London, UK ISSN: 2044-6055, DOI: 10.1136/bmjopen-2022-065777 Retrieved from the Internet: URL:https://findresearcher.sdu.dk/ws/portalfiles/portal/224299723/e065777.full.pdf> | 1,3-8, 13-18 | |
| Y | * page 1 - page 6 * | 1,3-11, 13-18 | **TECHNICAL FIELDS SEARCHED (IPC)** H04R |
| Y | EP 3 934 279 A1 (OTICON AS [DK]) 5 January 2022 (2022-01-05) * paragraph [0081] - paragraph [0114]; figures 7, 8B * | 1,3-8, 12-17 | |
| Y | SANCHEZ-LOPEZ RAUL: "IHCON 2022 International Hearing Aid Seminar (IHAS)", INTERNATIONAL HEARING AID RESEARCH CONFERENCE, vol. 2, 14 August 2022 (2022-08-14), XP093018713, * paragraph [0053] - paragraph [0054] * | 2 | |

-/--

The present search report has been drawn up for all claims

| Place of search | Date of completion of the search | Examiner |
|---|---|---|
| Munich | 30 April 2025 | Duffner, Orla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

**Application Number**

EP 25 15 4843

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EDRAKI AMIN ET AL: "Speech Intelligibility Prediction Using Spectro-Temporal Modulation Analysis", ARXIV:1806.04885V2,, vol. 29, 24 November 2020 (2020-11-24), pages 210-225, XP011824134, DOI: 10.1109/TASLP.2020.3039929 [retrieved on 2020-12-09] * page 210 - page 216 * ----- | 9-12 | |

TECHNICAL FIELDS SEARCHED (IPC)

The present search report has been drawn up for all claims

| Place of search | Date of completion of the search | Examiner |
|---|---|---|
| Munich | 30 April 2025 | Duffner, Orla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.........................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 15 4843

This annex lists the patent family members relating to the patent documents cited in the above-mentioned European search report.
The members are as contained in the European Patent Office EDP file on
The European Patent Office is in no way liable for these particulars which are merely given for the purpose of information.

30-04-2025

| Patent document cited in search report | Publication date | Patent family member(s) | Publication date |
|---|---|---|---|
| US 2023037356 A1 | 09-02-2023 | EP 4132010 A2 | 08-02-2023 |
| | | US 2023037356 A1 | 09-02-2023 |
| EP 3934279 A1 | 05-01-2022 | CN 113891225 A | 04-01-2022 |
| | | EP 3934279 A1 | 05-01-2022 |
| | | US 2022007116 A1 | 06-01-2022 |

For more details about this annex : see Official Journal of the European Patent Office, No. 12/82

**REFERENCES CITED IN THE DESCRIPTION**

**Non-patent literature cited in the description**

- **ZAAR, J** ; **SIMONSEN, L. B** ; **BEHRENS, T.** ; **DAU, T** ; **LAUGESEN, S.** Investigating the relationship between spectro-temporal modulation detection, aided speech perception, and directional noise reduction preference in hearing-impaired listeners. *Proceedings of the International Symposium on Auditory and Audiological Research*, 2019, vol. 7, 181-188

- **CUBICK, J.** ; **CAPORALI, S.** ; **LELIC, D** ; **CATIC, J.** ; **VIKÆR DAMSGAARD, A.** ; **ROSE, S.** ; **IVES, T.** ; **SCHMIDT, E**. The Acoustics of Instant Ear Tips and Their Implications for Hearing-Aid Fitting.. *Ear & Hearing*, 2022, vol. 43 (6), 1771-1782
- **CARLE, R** ; **LAUGESEN, S** ; **NIELSEN, C**. Observations on the Relations among Occlusion Effect, Compliance, and Vent Size.. *J. Am. Acad. Audiol.*, 2002, vol. 13 (1), 25-37